# EUROPEAN PATENT APPLICATION

(11) **EP 4 560 015 A2**
(43) Date of publication of application: **28.05.2025**
(21) Application number: 24215851.7
(22) Date of filing: 27.11.2024
(51) Int. Cl.: C12N 9/10, C12N 1/20, C12P 7/26, C12N 15/52, C12R 1/40

(54) **MODIFIED POLYKETIDE SYNTHASE ENZYMES AND USES THEREOF**

(30) Priority: 27.11.2023 EP 23307060
(71) Applicant: BGene Genetics, 38000 Grenoble (FR)
(72) Inventor: GOUSSE, Matthieu, 38000 GRENOBLE (FR); DE ANDRADE, Ricardo, 38000 GRENOBLE (FR); CHANDOR-PROUST, Alexia, 38000 GRENOBLE (FR)
(74) Representative: Plasseraud IP

(57) **Abstract**

The present invention is in the field of genetically modified enzyme and microorganism comprising such a modified enzyme for the production of raspberry ketone or zingerone. The modified enzyme is a polyketide synthase (PKS) issued or derived from a wild type PKS. The modified PKS has the capability to produce vanillylidene acetone from feruloyl-CoA and/or 4-hydroxybenzalacetone from 4-coumaroyl-CoA in a more effective way as compared to wild type BAS or wild type PmPKS, in particular in recombinant bacteria strains.

## Description

### Technical field

The present invention is in the field of genetically modified enzyme and microorganism comprising such a modified enzyme for the production of zingerone and/or raspberry ketone. The modified enzyme is a recombinant polyketide synthase (PKS) issued or derived from a wild type PKS. The modified PKS has the capability to produce vanillylidene acetone from feruloyl-CoA, and/or 4-hydroxybenzalacetone from coumaroyl-CoA in a more effective way as compared to wild type benzalacetone synthase (BAS) or wild type polyketide synthase (PKS).

### Prior art

The bioproduction of "natural" flavors and fragrances has been an important area of research for the industry for many years in order to meet the needs of consumers who are increasingly concerned about being environmentally responsible. Synthetic biology, in particular through the use of microorganisms, allows this natural production, but the yields are not always sufficient for large-scale production.

The flavor of raspberry (*Rubus idaeus*) is linked to more than 200 compounds, but raspberry ketone (also known under frambinone), a naturally occurring phenolic compound, is the most impactful compound, defining its characteristic flavor (Klesk et al., 2004, J. Agric. Food Chem. 52, 5155- 61; Larsen et al., 1991, Acta Agric. Scand. 41, 447-54).

Since it is present only in small amounts in raspberries (1-4 mg per kg of fruit), natural raspberry ketone is of great value (Larsen et al., 1991). However, because its natural availability is limited, its biotechnological production is highly desirable. However, raspberry flavor is a major ingredient for flavor and flagrance industry and is thus very much in demand.

Raspberry ketone is a polyketide. Polyketides represent a large family of metabolites produced by plants. Some of them are widely used in medicine, agriculture, and cosmetic industry. Flavonoids, stilbenes, benzalacetones, are polyketides.

Zingerone, also called vanillylacetone, is a major flavor component of ginger (Zingiber officinale). Zingerone is similar in chemical structure to other flavor chemicals such as vanillin, eugenol or raspberry ketone. It is used as a flavor additive in spice oils and in perfumery to introduce spicy aromas.

The current method for synthesizing zingerone involves chemistry reaction between vanillin and acetone under basic conditions to form dehydrozingerone. This reaction is followed by catalytic hydrogenation of the intermediate compound in order to form zingerone.

Thus, it is possible to produce synthetic flavors like zingerone or raspberry ketone, but biosynthesis is nowadays preferable and especially gives a natural molecule unlike the petrochemical production route.

In this context, the biosynthetic pathway of phenylpropanoid compounds, can be reconstituted within a microorganism through the insertion of heterologous genes encoding certain key enzymes of said pathway, thereby producing raspberry ketone in recombinant microorganism.

The raspberry ketone and zingerone production pathways follow the phenylpropanoid production pathway in plants. Phenylpropanoids are key molecules for flavor production in most plants. The phenylpropanoid biosynthetic pathway can be reconstituted within a microorganism by inserting heterologous plant genes encoding some key enzymes of the pathway such as Tyrosine Ammonia Lyase ("TAL", catalyzing the transformation of Tyrosine into coumaric acid) and polyketide Synthases ("PKS", catalyzing the transformation of cinnamoyl-CoA derivatives into benzalacetones. These benzalacetones can thereafter be reduced by reductase enzymes to synthesize aromatic compounds such as zingerone and raspberry ketone.

For producing zingerone, ferulic acid is converted into feruloyl-CoA by a 4-coumarate-ligase. Feruloyl-CoA is thereafter converted into vanillylidene acetone (or "VDA") by a type III polyketide synthase with benzalacetone synthase (BAS) activity. VDA is then reduced into zingerone by a benzalacetone reductase enzyme (or "BAR").

The production of raspberry ketone is made from p-coumaroyl-CoA by a 2-step reaction catalyzed by a type III polyketide synthase with benzalacetone synthase (BAS) activity. This BAS enzyme catalyzes the condensation of a p-coumaroyl-CoA molecule with a malonyl-CoA molecule into a 4-hydroxybenzalacetone (Abe et al., 2001). The 4-hydroxybenzalacetone molecule (or "4-HBA") is then reduced to raspberry ketone by a benzalacetone reductase enzyme (or "BAR"). This pathway was recently implemented in the yeast Saccharomyces cerevisiae and resulted in the de novo production of raspberry ketone (Lee et al., 2016).

To sum up, Benzalacetone Synthases ("BAS" or "BAS enzyme") catalyze the transformation of feruloyl-CoA into vanillylidene acetone or of coumaroyl-CoA into hydroxybenzalacetone, and vanillylidene acetone and hydroxybenzalacetone are then metabolized to zingerone and raspberry ketone, respectively, by a benzalacetone reductase enzyme such as one issued from the raspberry Rubus idaeus (RZS/RKS).

A BAS enzyme exists in raspberry but BAS enzyme from *Rheum palmatum* (rhubarb) is the most commonly used in synthetic biology.

So far, the BAS enzymes from *Rheum palmatum, Wachendorfia thyrsiflora, Rubus ideaus* and *Polygonum cuspidatum* have been cloned and tested (Shimokawa et al., 2012). However, in all microorganisms where the zingerone or raspberry ketone biosynthetic pathway has been implemented, the BAS enzyme from rhubarb (*Rheum palmatum*) is the one preferred (Wang et al., 2019; Milke et al., 2020; Chang et al., 2021).

Among the different studies on the activity of BAS, different analyses have been performed on the catalytic constant during the enzymatic reaction catalyzed by the BAS (Km affinity and kcat catalytic constant). For coumaroyl-CoA, kcat = 1.79 min-1 and affinity KM = 10.0 µM and for malonyl-CoA kcat = 1.78 min-1 and KM = 23.3 µM. Thus, this enzyme is rather slow, which poses a problem of metabolic flux when inserting it into a biosynthetic pathway for biotechnological purposes (Abe et al., 2001; 2007; Morita et al., 2010).

However, this BAS from *Rheum palmatum* is not very efficient, and may not be compatible with an efficient production of zingerone or raspberry ketone in fermentation. The development of more efficient process is crucial to produce flavor compounds such as raspberry ketone or zingerone or others in microorganisms. The catalytic constants of raspberry or rhubarb BAS enzymes are very low compared to other enzymes in the bioproduction pathway of raspberry ketone. The carbon flow is therefore slowed down by this enzyme, which does not allow an efficient conversion of the precursors (including feruloyl-CoA or coumaroyl-CoA) into benzalacetone derivatives (including vanillylidene acetone and 4-hydroxybenzalacetone and/or zingerone and raspberry ketone). Having more efficient transformation of including feruloyl-CoA or coumaroyl-CoA will lead to the provision of more efficient microorganism for producing flavor compounds such as raspberry ketone or zingerone. Having more efficient enzyme will lead to the provision of enhanced processes for producing phenylpropanoid compounds, including vanillylidene acetone, and/or 4-hydroxybenzalacetone compound and/or zingerone and/or raspberry ketone. Such an improved process would be more efficient and economically profitable with increased production yields of the final product raspberry ketone or zingerone.

Thus, there is still a need to develop new enzymes and new microorganisms to produce phenylpropanoid compounds, including vanillylidene acetone and/or 4-hydroxybenzalacetone and/or zingerone and/or raspberry ketone.

The object of the present invention is thus to provide modified enzymes, in particular modified enzymes that catalyze the transformation of feruloyl-CoA into vanillylidene acetone and/or of coumaroyl-CoA into 4-hydroxybenzalacetone. These modified enzymes are PKS that have an enhanced catalytic activity as compared to wild type enzymes that transform feruloyl-CoA into vanillylidene acetone and/or of coumaroyl-CoA into 4-hydroxybenzalacetone.

One of the aims of the invention is to provide a modified PKS enzyme and a genetically modified microorganism comprising means for expressing such a modified PKS enzyme allowing a more efficient production of vanillylidene acetone and/or 4-hydroxybenzalacetone compound and/or zingerone and/or raspberry ketone, in particular from a feruloyl-CoA or from a coumaroyl-CoA. In particular, there is a need for an enzyme that enhances the conversion of feruloyl-CoA into vanillylidene acetone and/or of coumaroyl-CoA into benzalacetone 4-hydroxybenzalacetone, as compared to the enzymes used in the prior art.

### Summary of the invention

In a first aspect of the present invention, it is provided a recombinant polyketide synthase (PKS) issued or derived from a wild type PKS from *Piper methysticum* (PmPKS), *Elaeis guineensis* (EgPKS), or *Vitis vinifera* (VvPKS), the recombinant PKS having a P38A substitution, and/or a V322P substitution as compared to the wild type PKS from *Piper methysticum,* or the recombinant PKS having a P39A substitution as compared to the wild type PKS from *Elaeis guineensis,* or the recombinant PKS having a P37A substitution and/or a M64Q substitution as compared to the wild type PKS from *Vitis vinifera* In another aspect of the present invention, it is provided a recombinant microorganism, in particular a recombinant *Pseudomonas putida,* capable to express or expressing a recombinant PKS enzyme according to the invention. A recombinant PKS can also be referenced as a "modified PKS".

In another aspect, it is provided a genetically modified microorganism, in particular a recombinant microorganism, more particularly a recombinant *Pseudomonas putida,* capable to express or expressing a recombinant PKS enzyme according to the invention for the production of phenylpropanoid compounds, in particular vanillylidene acetone and/or 4-hydroxybenzalacetone and/or zingerone and/or raspberry ketone, more particularly vanillylidene acetone and/or zingerone.

In another aspect, it is provided a recombinant PKS enzyme or a genetically modified microorganism, in particular a recombinant microorganism, more particularly a recombinant *Pseudomonas putida* capable of expressing or expressing a recombinant PKS enzyme according to the invention, for use in the production of phenylpropanoid compounds, in particular vanillylidene acetone and/or 4-hydroxybenzalacetone and/or zingerone and/or raspberry ketone, more particularly vanillylidene acetone and/or zingerone, more particularly vanillylidene acetone and/or zingerone.

In another aspect, it is provided a process for the synthesis of phenylpropanoid compounds, vanillylidene acetone and/or 4-hydroxybenzalacetone and/or zingerone and/or raspberry ketone, more particularly vanillylidene acetone and/or zingerone, more particularly vanillylidene acetone and/or zingerone, by using a PKS enzyme according to the invention, and/or by using a genetically modified microorganism, in particular a recombinant microorganism, more particularly a recombinant *Pseudomonas putida,* capable to express or expressing a recombinant PKS enzyme according to the invention.

The invention is defined in the independent claims. Dependent claims define preferred embodiments. The features set forth in the following paragraphs may optionally be implemented. They can be implemented independently of each other or in combination with each other.

### Short description of the drawings

These and further aspects of the invention will be explained in greater detail by way of examples and with reference to the accompanying drawings in which:
**Fig. 1**

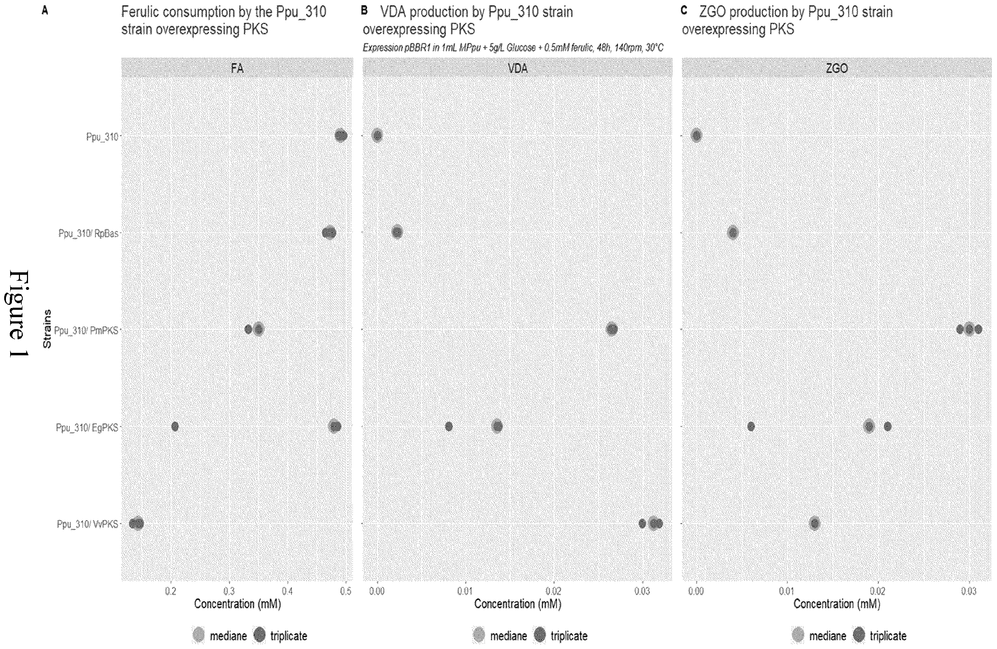

   Fig. 1 is a graph illustrating the concentration of (**A**) ferulic acid, (**B**) vanillylidene acetone (VDA) and (**C**) zingerone (ZGO) in supernatants of cultured *P. putida.* The abscissa axis represents the concentration in mM of ferulic acid (Fig. 1, left side), VDA (Fig. 1, middle) and ZGO (Fig. 1, right side). On the ordinate axis, microorganisms comprising different wild type polyketide synthases issued from *Piper methysticum, Elaeis guineensis, Vitis vinifera* or a wild type BAS issued from *Rheum palmatum* are identified. Ppu_310 corresponds to control microorganisms that do not express recombinant PKS or BAS.
**Fig. 2**

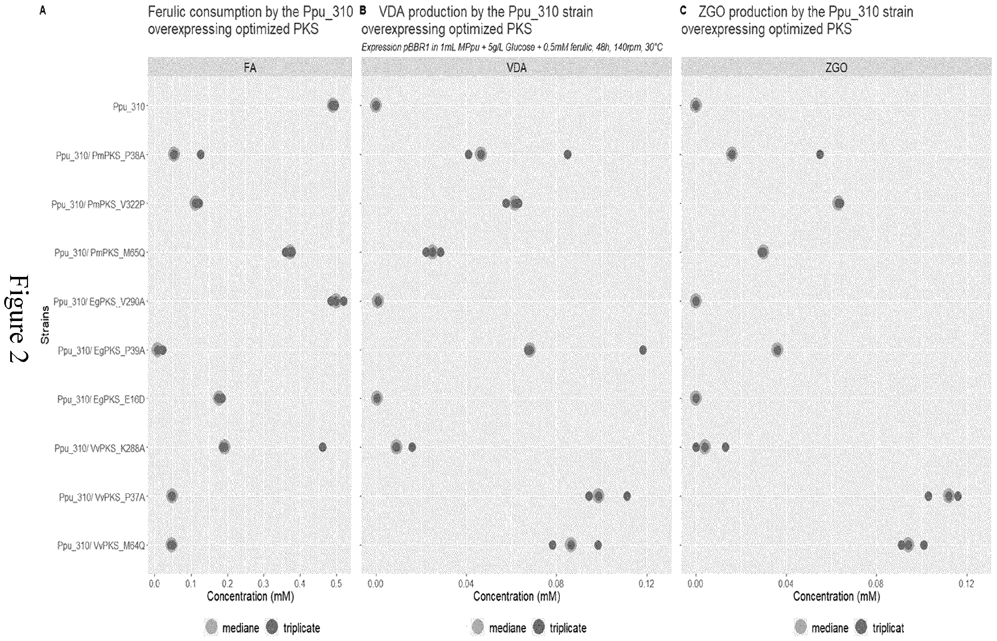

   Fig. 2 is a graph illustrating the concentration of (**A**) ferulic acid, (**B**) vanillylidene acetone (VDA) and (**C**) zingerone (ZGO) in supernatants of cultured recombinant *P*. *putida.* Recombinant *Pseudomonas putida* expressing modified PKS are identified on the ordinate axis. Abscissa axis corresponds to the concentration of **A**) ferulic acid, (**B**) vanillylidene acetone (VDA) and (**C**) zingerone (ZGO) expressed in mM in the supernatant of cultured *Pseudomonas putida.*
**Fig. 3**

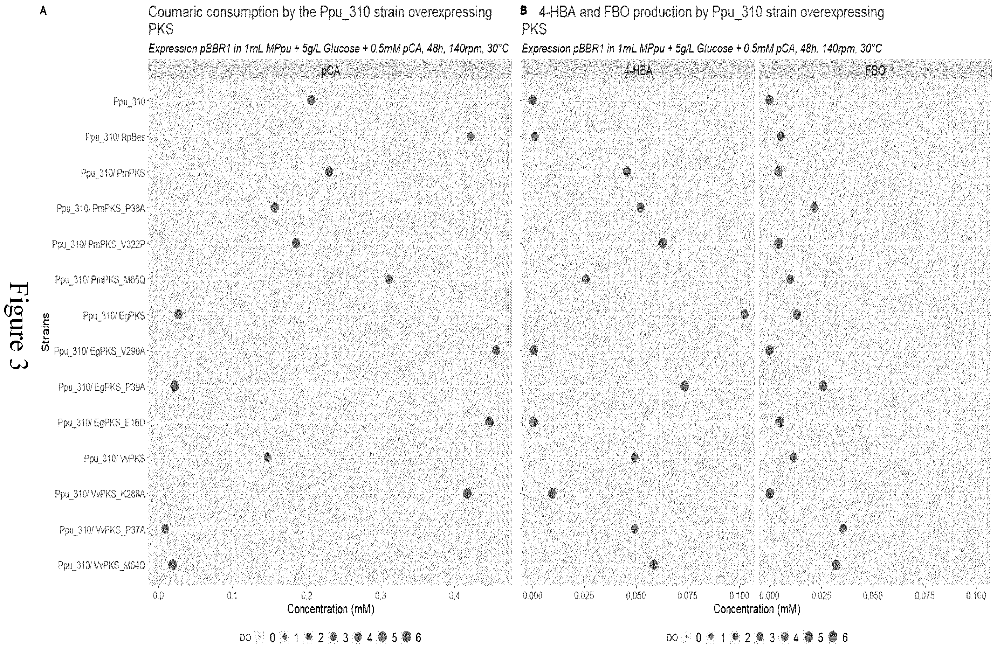

   Fig. 3 is a graph illustrating the concentration of p-Coumaric acid (pCA), 4-hydroxybenzalacetone (HBA) and raspberry ketone (FBO) in supernatants of cultured *P. putida.* The abscissa axis represents the concentration in mM of pCA (Fig. 1, left side), HBA (Fig. 1, middle) and FBO (right side). On the ordinate axis, different PKS enzymes are listed, wherein the modified PKS are defined by their mutation as compared to the wild-type PKS from which they are derived. Ppu_310 corresponds to control microorganisms that do not express recombinant PKS or BAS. RpBAs corresponds to the BAS from *Rheum palmatum.*

### Detailed description of embodiments of the invention

In a first aspect, it is provided a recombinant polyketide synthase (PKS) issued or derived from a wild type PKS from *Piper methysticum* (PmPKS), *Elaeis guineensis* (EgPKS), or *Vitis vinifera* (VvPKS), the recombinant PKS having a P37A substitution and/or a M64Q substitution as compared to the wild type PKS from *Vitis vinifera,* or the recombinant PKS having a P38A substitution and/or a V322P substitution as compared to the wild type PKS from *Piper methysticum,* or the recombinant PKS having a P39A substitution as compared to the wild type PKS from *Elaeis guineensis.*

Such a recombinant PKS may have the capability to convert feruloyl-CoA into vanillylidene acetone. Such a modified PKS may convert feruloyl-CoA into vanillylidene acetone in a more efficient manner than a wild type BAS issued from *Rheum palmatum,* and/or than the wild type PKS from which it is issued or derived.

Such a recombinant PKS may have the capability to convert p-coumaroyl-CoA into 4-hydroxybenzalcetone. Such a recombinant PKS may convert p-coumaroyl-CoA into 4-hydroxybenzalcetone in a more efficient manner than a wild type BAS issued from *Rheum palmatum,* and/or than the wild type PKS from which it is issued or derived.

Polyketide synthases are a family of multi-domain enzymes or enzyme complexes that produce polyketides. In a particular embodiment of the invention, the recombinant PKS and the wild type PKS are type III polyketide synthase with benzalacetone synthase (BAS) activity. Type III PKSs are small homodimers of 40 kDa proteins that may combine several or all the activities from the essential type I and II PKS domains (e.g., acyltransferase, keto synthase, thiolation, keto reductase or dehydratase. However, in contrast to type I and II PKSs they do not require an ACP-bound substrate. Instead, they can use a free acyl-CoA substrate for chain elongation. Type III PKSs contain a Cys-His-Asn catalytic triad in their active center, with the cysteine residue acting as the attacking nucleophile, whereas type I and II PKSs are characterized by a Cys-His-His catalytic triad. In an embodiment of the invention, the wild type PKS from which is issued the recombinant PKS of the invention are type III polyketide synthase from *Piper methysticum, Elaeis guineensis,* or *Vitis vinifera.*

In a particular embodiment of the invention, the recombinant PKS has an improved catalytic activity on the transformation of feruloyl-CoA into vanillylidene acetone as compared to the wild type PKS from which it is issued or derived.

In a particular embodiment of the invention, the recombinant PKS has an improved catalytic activity on the transformation of coumaroyl-CoA into 4-hydroxybenzalacetone as compared to the wild type PKS from which it is issued or derived.

The recombinant PKS may be obtained by genetic engineering of the wild type PKS from which it is issued or derived, or from a gene or nucleic acid molecule encoding such a wild type PKS. One or more non-natural mutations can be introduced into the recombinant PKS, for example by insertion, substitution or deletion of nucleotides encoding the wild type PKS, said mutations being obtained by transformation techniques or by gene editing techniques known to the skilled artisan.

Genetic modification techniques by transformation, mutagenesis or gene editing are described for example in "Strategies used for genetically modifying bacterial genome: site-directed mutagenesis, gene inactivation", Journal of Zhejiang Univ-Sci B (Biomed & Biotechnol) 2016 17(2):83-99., and in Martinez-Garcia and de Lorenzo, "Pseudomonas putida in the quest of programmable chemistry", Current Opinion in Biotechnology, 59 :111-121, 2019.

In an aspect of the invention, the wild type PKS from which the recombinant PKS is issued or derived is from *Vitis vinifera* (the enzyme may be identified as VvPKS), or from *Piper methysticum* (the enzyme may be identified as PmPKS), or from *Elaeis guineensis* (the enzyme may be identified as EgPKS). In a particular embodiment, the wild type PKS from which the recombinant PKS is issued or derived is from *Vitis vinifera.* In a particular embodiment, the wild type PKS from which the recombinant PKS is issued or derived is from *Piper methysticum.* In a particular embodiment, the wild type PKS from which the recombinant PKS is issued or derived is from *Elaeis guineensis.*

More particularly, the wild type PKS from which the recombinant PKS is issued or derived has the amino acid sequence set forth in SEQ ID No. 3, that corresponds to wild type PKS from *Vitis vinifera.*

More particularly, the wild type PKS from which the recombinant PKS is issued or derived has the amino acid sequence set forth in SEQ ID No. 1, that corresponds to wild type PKS from *Piper methysticum.*

More particularly, the wild type PKS from which the recombinant PKS is issued or derived has the amino acid sequence set forth in SEQ ID No. 2, that corresponds to wild type PKS from *Elaeis guineensis.*

In an embodiment, the wild type PKS is encoded by the nucleotide sequence set forth in SEQ ID No. 16 or SEQ ID No. 17 or SEQ ID No. 18. In an embodiment, the PKS (either wild type or the recombinant one) is encoded within a vector, in particular a plasmid, having the nucleotide sequence set forth in SEQ ID No. SEQ ID No. 34, or SEQ ID No. 38 or SEQ ID No. 43.

By issued or derived, it should be understood that the recombinant PKS of the invention is modified as compared to the wild type PKS by mutation, including by substitution (including conservative amino acid residue(s)) or by addition and/or deletion of amino acid residues or by secondary modification after translation or by deletion of portions of the wild type PKS resulting in fragments having a shortened size with respect to the wild type PKS of reference. Fragments of the wild type PKS are encompassed within the present invention to the extent that they possess the capability to transform feruloyl-CoA into vanillylidene acetone, and/or the capability to transform coumaroyl-CoA into 4-hydroxybenzalacetone, and the P38A substitution and/or a V322P substitution as compared to the wild-type PKS from *Piper methysticum,* or the P39A substitution as compared to the wild-type PKS from *Elaeis guineensis,* or the P37A substitution and/or a M64Q substitution as compared to the wild-type PKS from *Vitis vinifera.*

In particular, a recombinant PKS may be obtained by modifying the amino acid sequence of wild type PKS by the method detailed in example 1.

In a particular aspect of the invention, the recombinant PKS of the invention has an improved catalytic activity on the transformation of feruloyl-CoA into vanillylidene acetone as compared to the catalytic activity of the wild type BAS from *Rheum palmatum* on the same transformation, and/or to the catalytic activity of the wild type PKS from *Piper methysticum* on the same transformation.

In a more particular aspect, the recombinant PKS of the invention improves at least 2, 3, 4, 5, 6 or 7 times more the transformation of feruloyl-CoA into vanillylidene acetone as compared to the catalytic activity of the wild type BAS from *Rheum palmatum* on the same transformation, and/or to the catalytic activity of the wild type PKS from *Piper methysticum* on the same transformation.

The catalytic activity of the recombinant PKS may be determined by measuring the catalytic constant k_{cat} of the PKS in a transformation reaction of feruloyl-CoA into vanillylidene acetone. It can be calculated from the maximum reaction rate and catalyst site concentration by methods known by the skilled artisan.

In a particular embodiment, the recombinant PKS of the invention has at least the same catalytic activity, in particular at least the same catalytic constant k_{cat}, on the transformation reaction of feruloyl-CoA into vanillylidene acetone, as compared to the wild type BAS from *Rheum palmatum,* in particular the wild type BAS having the amino acid sequence set forth in SEQ ID No. 9, and/or as compared to the wild type PKS from *Piper methysticum,* in particular the PKS having the amino acid sequence set forth in SEQ ID No. 1.

In a particular aspect of the invention, the recombinant PKS of the invention has an improved catalytic activity on the transformation of coumaroyl-CoA into a 4-hydroxybenzalacetone as compared to the catalytic activity of the wild type BAS from *Rheum palmatum* on the same transformation, and/or to the catalytic activity of the wild type PKS from *Piper methysticum* on the same transformation.

In a more particular aspect, the recombinant PKS of the invention improves at least 2, 3, 4, 5, 6 or 7 times more the transformation of coumaroyl-CoA into a 4-hydroxybenzalacetone as compared to the catalytic activity of the wild type BAS from *Rheum palmatum* on the same transformation, and/or to the catalytic activity of the wild type PKS from *Piper methysticum* on the same transformation.

The catalytic activity of the recombinant PKS may be determined by measuring the catalytic constant k_{cat} of the PKS in a transformation reaction of coumaroyl-CoA into a 4-hydroxybenzalacetone. It can be calculated from the maximum reaction rate and catalyst site concentration by methods known by the skilled artisan.

In a particular embodiment, the recombinant PKS of the invention has at least the same catalytic activity, in particular at least the same catalytic constant k_{cat}, on the transformation reaction of coumaroyl-CoA into a 4-hydroxybenzalacetone, as compared to the wild type BAS from *Rheum palmatum,* in particular the wild type BAS having the amino acid sequence set forth in SEQ ID No. 9, and/or as compared to the wild type PKS from *Piper methysticum,* in particular the PKS having the amino acid sequence set forth in SEQ ID No. 1.

In an embodiment of the invention, the recombinant PKS of the invention has a P37A substitution as compared to the wild type PKS from *Vitis vinifera,* in particular as compared to the wild type PKS from *Vitis vinifera* of SEQ ID No. 3, more particularly the PKS of the invention has the amino acid sequence set forth in SEQ ID No. 7.

In an embodiment of the invention, the recombinant PKS of the invention has a M64Q substitution as compared to the wild type PKS from *Vitis vinifera,* in particular as compared to the wild type PKS from *Vitis vinifera* of SEQ ID No. 3, more particularly the PKS of the invention has the amino acid sequence set forth in SEQ ID No. 8.

In an embodiment of the invention, the recombinant PKS of the invention has a P37A substitution and a M64Q substitution as compared to the wild type PKS from *Vitis vinifera,* in particular as compared to the wild type PKS from *Vitis vinifera* of SEQ ID No. 3, more particularly the PKS of the invention has the amino acid sequence set forth in SEQ ID No. 11.

In an embodiment of the invention, the recombinant PKS of the invention has a P38A substitution as compared to the wild type PKS from *Piper methysticum,* in particular as compared to the wild type PKS from *Piper methysticum* of SEQ ID No. 1, more particularly the PKS of the invention has the amino acid sequence set forth in SEQ ID No. 4.

In an embodiment of the invention, the recombinant PKS of the invention has a V322P substitution as compared to the wild type PKS from *Piper methysticum,* in particular as compared to the wild type PKS from *Piper methysticum* of SEQ ID No. 1, more particularly the PKS of the invention has the amino acid sequence set forth in SEQ ID No. 5.

In an embodiment of the invention, the recombinant PKS of the invention has a P38A substitution and a V322P as compared to the wild type PKS from *Piper methysticum,* in particular as compared to the wild type PKS from *Piper methysticum* of SEQ ID No. 1, more particularly the PKS of the invention has the amino acid sequence set forth in SEQ ID No. 10.

In an embodiment of the invention, the recombinant PKS of the invention has a P39A substitution as compared to the wild type PKS from *Elaeis guineensis,* in particular as compared to the wild type PKS from *Elaeis guineensis* of SEQ ID No. 2, more particularly the PKS of the invention has the amino acid sequence set forth in SEQ ID No. 6.

In an embodiment of the invention, the recombinant PKS of the invention has an amino acid sequence selected from the sequences set forth in the group consisting of SEQ ID No. 7; SEQ ID No. 8; SEQ ID No. 11; SEQ ID No. 4; SEQ ID No. 5; SEQ ID No. 6 and SEQ ID No. 10.

In an embodiment of the invention, the recombinant PKS of the invention has an amino acid sequence which has at least 90% identity, more preferably at least 91%, more preferably at least 92%, more preferably at least 93%, more preferably at least 94%, more preferably at least 95%, more preferably at least 96%, more preferably at least 97%, more preferably at least 98%, more preferably at least 99%, with the PKS having the amino acid sequence set forth in SEQ ID No. 4.

In an embodiment of the invention, the recombinant PKS of the invention has an amino acid sequence which has at least 90% identity, more preferably at least 91%, more preferably at least 92%, more preferably at least 93%, more preferably at least 94%, more preferably at least 95%, more preferably at least 96%, more preferably at least 97%, more preferably at least 98more preferably at least 99%, with the PKS having the amino acid sequence set forth in SEQ ID No. 5.

In an embodiment of the invention, the recombinant PKS of the invention has an amino acid sequence which has at least 90% identity, more preferably at least 91%, more preferably at least 92%, more preferably at least 93%, more preferably at least 94%, more preferably at least 95%, more preferably at least 96%, more preferably at least 97%, more preferably at least 98%, more preferably at least 99%, with the PKS having the amino acid sequence set forth in SEQ ID No. 6.

In an embodiment of the invention, the recombinant PKS of the invention has an amino acid sequence which has at least 90% identity, more preferably at least 91%, more preferably at least 92%, more preferably at least 93%, more preferably at least 94%, more preferably at least 95%, more preferably at least 96%, more preferably at least 97%, more preferably at least 98%, more preferably at least 99%, with the PKS having the amino acid sequence set forth in SEQ ID No. 7.

In an embodiment of the invention, the recombinant PKS of the invention has an amino acid sequence which has at least 90% identity, more preferably at least 91%, more preferably at least 92%, more preferably at least 93%, more preferably at least 94%, more preferably at least 95%, more preferably at least 96%, more preferably at least 97%, more preferably at least 98%, more preferably at least 99%, with the PKS having the amino acid sequence set forth in SEQ ID No. 8.

In an embodiment of the invention, the recombinant PKS of the invention has an amino acid sequence which has at least 90% identity, more preferably at least 91%, more preferably at least 92%, more preferably at least 93%, more preferably at least 94%, more preferably at least 95%, more preferably at least 96%, more preferably at least 97%, more preferably at least 98%, more preferably at least 99%, with the PKS having the amino acid sequence set forth in SEQ ID No. 10.

In an embodiment of the invention, the recombinant PKS of the invention has an amino acid sequence which has at least 90% identity, more preferably at least 91%, more preferably at least 92%, more preferably at least 93%, more preferably at least 94%, more preferably at least 95%, more preferably at least 96%, more preferably at least 97%, more preferably at least 98%, more preferably at least 99%, with the PKS having the amino acid sequence set forth in SEQ ID No. 11.

In a particular embodiment, the recombinant PKS of the invention is issued or derived from a wild type PKS, the recombinant PKS having a P39A substitution as compared to the wild type PKS from *Piper methysticum* and is a functional equivalent of the PKS having the amino acid sequence set forth in SEQ ID No. 4 or SEQ ID No. 5.

In a particular embodiment, the recombinant PKS of the invention is issued or derived from a wild type PKS, the recombinant PKS having a V322P substitution as compared to the wild type PKS from *Piper methysticum* and is a functional equivalent of the PKS having the amino acid sequence set forth in SEQ ID No. 4 or SEQ ID No. 5.

In a particular embodiment, the recombinant PKS of the invention is issued or derived from a wild type PKS, the recombinant PKS having a P38A substitution and a V322P substitution as compared to the wild type PKS from *Piper methysticum* and is a functional equivalent of the PKS having the amino acid sequence set forth in SEQ ID No. 4 or SEQ ID No. 5.

In a particular embodiment, the recombinant PKS of the invention is issued or derived from a wild type PKS, the recombinant PKS having a P39A substitution as compared to the wild type PKS from *Elaeis guineensis* and is a functional equivalent of the PKS having the amino acid sequence set forth in SEQ ID No. 6.

In a particular embodiment, the recombinant PKS of the invention is issued or derived from a wild type PKS, the recombinant PKS having a P37A substitution as compared to the wild type PKS from *Vitis vinifera* and is a functional equivalent of the PKS having the amino acid sequence set forth in SEQ ID No. 7 or SEQ ID No. 8.

In a particular embodiment, the recombinant PKS of the invention is issued or derived from a wild type PKS, the recombinant PKS having a M64Q substitution as compared to the wild type PKS from *Vitis vinifera* and is a functional equivalent of the PKS having the amino acid sequence set forth in SEQ ID No. 7 or SEQ ID No. 8.

In a particular embodiment, the recombinant PKS of the invention is issued or derived from a wild type PKS, the recombinant PKS having a P37A substitution and a M64Q substitution as compared to the wild type PKS from *Vitis vinifera* and is a functional equivalent of the PKS having the amino acid sequence set forth in SEQ ID No. 7 or SEQ ID No. 8.

A functional variant of the wild type PKS having the amino sequence set forth in SEQ ID No. 4, SEQ ID No. 5, SEQ ID No. 6, SEQ ID No. 7 or SEQ ID No. 8 is a PKS having a polypeptide sequence which is issued or derived from the polypeptide sequence of one wild type PKS, in particular a wild type PKS from *Piper methysticum, Elaeis guineensis* or *Vitis vinifera,* the functional variant may comprise further modification(s), i.e. substitution(s), insertion(s) and/or deletion(s) of one or more amino acids but which retains the activity of the PKS having the amino acid sequence set forth in SEQ ID No. 4, SEQ ID No. 5, SEQ ID No. 6, SEQ ID No. 7 or SEQ ID No. 8, and in particular the capability to convert feruloyl-CoA into vanillylidene acetone and/or coumaroyl-CoA into 4-hydroxybenzalcetone as described above with at least the same catalytic activity, with the proviso that the recombinant PKS still has the required substitution(s) as compared to the wild type protein from which it is issued. Fragments may have a size representing more than 50% of the amino-acid sequence size of the wild type PKS, more particularly more than 60%, more particularly more than 70%, more particularly more than 80%, more particularly more than 90%, and most particularly most than 95%.

The catalytic activity of a functional equivalent of the PKS having the amino acid sequence set forth in SEQ ID No. 4, SEQ ID No. 5, SEQ ID No. 6, SEQ ID No. 7 or SEQ ID No. 8 can be assessed by any method known to the skilled person, in particular as illustrated in the example of the invention, by expressing in a strain of *Pseudomonas putida* a recombinant gene encoding the functional variant of the PKS having the amino acid sequence set forth in SEQ ID No. 4, SEQ ID No. 5, SEQ ID No. 6, SEQ ID No. 7 or SEQ ID No. 8, preferably cloned into a plasmid downstream of a promoter allowing its expression in the strain, and culturing the strain in the presence of ferulic acid or feruloyl-CoA, or in the presence of coumaric acid or coumaroyl-CoA, and assaying by HPLC the total concentration of vanillylidene acetone and/or zingerone or 4-hydroxybenzalcetone and/or raspberry ketone, preferably vanillylidene acetone and/or zingerone, produced by the strain after 24h. The functional variant maintains at least the same activity as compared to the PKS activity measured when the PKS having the amino acid sequence set forth in SEQ ID No. 4, SEQ ID No. 5, SEQ ID No. 6, SEQ ID No. 7 or SEQ ID No. 8is expressed in the strain of *Pseudomonas putida* in the same experimental condition. The PKS activity can be determined according to the amount or concentration of feruloyl-CoA and/or vanillylidene acetone and/or coumaroyl-CoA and/or 4-hydroxybenzalcetone and/or raspberry ketone and/or zingerone, preferably feruloyl-CoA and vanillylidene acetone or coumaroyl-CoA and 4-hydroxybenzalcetone before and after a catalytic reaction of transforming feruloyl-CoA into vanillylidene acetone and/or on the transformation of coumaroyl-CoA into 4-hydroxybenzalacetone.

PKS activity may be determined by measuring the quantity of vanillylidene acetone by HPLC in the following test:
PKS activity may be determined by measuring the quantity of vanillylidene acetone by HPLC in the following test:
In a 200µl medium comprising:
   - 0.5mM of Ferulic acid;1mM of coenzyme A,
   - 5mM of ATP,
   - 25mM of MgCl₂,
   - 1mM of malonyl-CoA,
   - 500mM of Tris-HCl buffer at pH 7.5,
   - 50µg/mL of 4-coumarate--CoA ligase 1,
50µg/ml of the PKS to be tested is added.
The medium with the PKS is incubated during 24 hours at 30°C.
After this 24h period, the enzymatic reaction is stopped by adding 50mM of HCl.
The production of vanillylidene acetone is measured by HPLC.
A PKS is considered to be a functional equivalent of the PKS of SEQ ID No. 4, SEQ ID No. 5, SEQ ID No. 6, SEQ ID No. 7 or SEQ ID No. 8 when the production of vanillylidene acetone in presence of the tested PKS is at least equal to the production of vanillylidene acetone in presence of the PKS of SEQ ID No. 4, SEQ ID No. 5, SEQ ID No. 6, SEQ ID No. 7 or SEQ ID No. 8.

PKS activity may be determined by measuring the quantity of 4-hydroxybenzalcetone by HPLC in the following test:
In a 200µl medium comprising:
   - 0.5mM of Coumaric acid;1mM of coenzyme A,
   - 5mM of ATP,
   - 25mM of MgCl₂,
   - 1mM of malonyl-CoA,
   - 500mM of Tris-HCl buffer at pH 7.5,
   - 50µg/mL of 4-coumarate--CoA ligase 1,
50µg/ml of the PKS to be tested is added.
The medium with the PKS is incubated during 24 hours at 30°C.
After this 24h period, the enzymatic reaction is stopped by adding 50mM of HCl.
The production of 4-hydroxybenzalcetone is measured by HPLC.
A PKS is considered to be a functional equivalent of the PKS of SEQ ID No. 4, SEQ ID No. 5, SEQ ID No. 6, SEQ ID No. 7 or SEQ ID No. 8 when the production of 4-hydroxybenzalcetone in presence of the tested PKS is at least equal to the production of 4-hydroxybenzalcetone in presence of the PKS of SEQ ID No. 4, SEQ ID No. 5, SEQ ID No. 6, SEQ ID No. 7 or SEQ ID No. 8.

Preferably, a functional equivalent of the PKS having the amino acid sequence set forth in SEQ ID No. 4, SEQ ID No. 5, SEQ ID No. 6, SEQ ID No. 7 or SEQ ID No. 8corresponds to a polypeptide sequence having at least 80%, 85%, 90%, 95% and most particularly, at least 98% identity with one of the sequences selected from SEQ ID No. 4, SEQ ID No. 5, SEQ ID No. 6, SEQ ID No. 7 or SEQ ID No. 8. A percentage identity refers to the percentage of identical residues in a nucleotide or amino acid sequence on a given fragment after alignment and comparison with a reference sequence. For the comparison, an alignment algorithm is used and the sequences to be compared are entered with the corresponding parameters of the algorithm. The default parameters of the algorithm can be used.

In a particular embodiment, for a nucleic acid or polypeptide sequence comparison and determination of a percent identity, the blastn or blastp algorithm as described in https://blast.ncbi.nlm.nih.gov/Blast.cgiavec is used, in particular with default parameters. In particular, the functional variant refers to a polypeptide that has an amino acid sequence that differs from one of the sequences selected from SEQ ID No. 4, SEQ ID No. 5, SEQ ID No. 6, SEQ ID No. 7 or SEQ ID No. 8 or SEQ ID No. 10 or SEQ ID No. 11 by less than 50, 40, 30, 20, 10, 5, 4, 3, 2 or 1 substitutions, insertions, or deletions.

In another particular embodiment, the functional variant refers to a polypeptide that has an amino acid sequence that differs from one of the sequences selected from SEQ ID No. 4, SEQ ID No. 5, SEQ ID No. 6, SEQ ID No. 7, SEQ ID No. 8, SEQ ID No. 10 or SEQ ID No. 11 by fewer than 50, 40, 30, 20, 10, 5, 4, 3, 2 or 1 substitutions, the substitutions preferably being conservative substitutions. The term "conservative substitution" as used herein refers to the replacement of one amino acid residue with another, without altering the conformation or enzymatic activity of the polypeptide so modified, including, but not limited to, the replacement of one amino acid with another having similar properties (such as, for example, polarity, hydrogen bonding potential, acidity, basicity, shape, hydrophobicity, aromaticity, and the like).

Examples of conservative substitutions are found in the groups of basic amino acids (arginine, lysine, and histidine), acidic amino acids (glutamic acid and aspartic acid), polar amino acids (glutamine and asparagine), hydrophobic amino acids (methionine, leucine, isoleucine, and valine), aromatic amino acids (phenylalanine, tryptophan, and tyrosine) and small amino acids (glycine, alanine, serine, and threonine).

In a particular embodiment, the wild type PKS enzyme is issued from *Piper methysticum* and has the amino acid sequence set forth in SEQ ID No. 1. In a particular embodiment, the wild type PKS enzyme is issued from *Elaeis guineensis* and has the amino acid sequence set forth in SEQ ID No. 2. In a particular embodiment, the wild type PKS enzyme is issued from *Vitis vinifera* and has the amino acid sequence set forth in SEQ ID No. 3.

In another embodiment, it is provided a nucleic acid molecule encoding a recombinant PKS according to the invention. In a preferred embodiment, the nucleic acid encodes the recombinant PKS according to any embodiment disclosed herein. In a particular embodiment, the nucleic acid molecule that encodes the recombinant PKS according to the invention has the sequence set forth in SEQ ID No. 19 or SEQ ID No. 20 or SEQ ID No. 21 or SEQ ID No. 22 or SEQ ID No. 23 or SEQ ID No. 25 or SEQ ID No. 26. In a particular embodiment, the nucleic acid molecule that encodes the recombinant PKS according to the invention is a plasmid, in particular is a plasmid comprising the sequence set forth in SEQ ID No. 19 or SEQ ID No. 20 or SEQ ID No. 21 or SEQ ID No. 22 or SEQ ID No. 23 or SEQ ID No. 25 or SEQ ID No. 26, or comprising or consisting of the sequence set forth in SEQ ID No. 35 or SEQ ID No. 36; or SEQ ID No. 40 or SEQ ID No. 41 or SEQ ID No. 45. The plasmid, or expression vector, may comprises or consists of the nucleotide sequence set forth in SEQ ID No. 31 or SEQ ID No. 33 or SEQ ID No. 42.

The invention also relates to a nucleic acid molecule encoding the recombinant PKS as disclosed herein or comprising the nucleic acid molecule(s) as disclosed herein. Encompassed within the invention is a vector encoding the recombinant PKS as disclosed herein. As used herein, a vector is a nucleic acid molecule used as a vehicle to transfer a genetic material into a cell, and in a preferred embodiment allows the expression of a gene encoding the recombinant PKS inserted within the vector. The term vector encompasses plasmids, viruses, cosmids and artificial chromosomes. The vector itself is generally a nucleotide sequence, commonly a DNA sequence, that comprises an insert (a transgene) and a larger sequence that serves as the "backbone" of the vector. Modern vectors may encompass additional features besides the transgene insert and a backbone: promoter, genetic marker, antibiotic resistance, reporter gene, targeting sequence, protein purification tag. Vectors called expression vectors (expression constructs) specifically are for the expression of the transgene (i.e., the gene encoding the recombinant PKS of the invention) in a target cell (i.e., a genetically engineered *Pseudomonas putida*)*,* and generally have control sequences.

In another embodiment of the invention, the applicant has developed a genetically modified strain of *Pseudomonas putida* capable of expressing a recombinant PKS to produce more efficiently vanillylidene acetone from feruloyl-CoA, and/or to produce more efficiently 4-hydroxybenzalketone from a coumaroyl-CoA according to the reaction as described above.

The recombinant PKS presents in the genetically modified strain of *Pseudomonas putida* capable of expressing the recombinant PKS of the invention is preferably any recombinant PKs as disclosed herein or is a wild type PKS from either *Piper methysticum, Elaeis guineensis,* or *Vitis vinifera.* In particular, the genetically modified strain of *Pseudomonas putida* is capable expressing a wild type PKS selected from the PKS having the amino acid sequence set forth in SEQ ID No. 1, SEQ ID No. 2, or SEQ ID No. 3, or a modified PKS having the amino acid sequence set forth in SEQ ID No. 4, SEQ ID No. 5, SEQ ID No. 6, SEQ ID No. 7, SEQ ID No. 8, SEQ ID No. 10 or SEQ ID No. 11, or a functional equivalent thereof, as defined here above.

According to another particular embodiment, the genetically modified *Pseudomonas putida* strain comprises an additional recombinant gene encoding a polypeptide with PKS activity. In particular, the modified strain may comprise a recombinant gene encoding PKS whose sequence is defined by the amino acid sequence SEQ ID No. 1, SEQ ID No. 2, SEQ ID No. 3, SEQ ID No. 4, SEQ ID No. 5, SEQ ID No. 6, SEQ ID No. 7, SEQ ID No. 8, SEQ ID No. 10 or SEQ ID No. 11, or by a sequence having at least 80%, 85%, 90%, 95%, 96%, 97%, 98%, and most particularly, at least 99% identity with the sequence SEQ ID No. 1, SEQ ID No. 2, SEQ ID No. 3, SEQ ID No. 4, SEQ ID No. 5, SEQ ID No. 6, SEQ ID No. 7, SEQ ID No. 8, SEQ ID No. 10 or SEQ ID No. 11 and encoding a PKS according to the invention.

According to a particular embodiment, the genetically modified *Pseudomonas putida* strain comprises an additional recombinant gene encoding a recombinant PKS comprising or having or consisting of the nucleotide sequence set forth in SEQ ID No. 16 or SEQ ID No. 17 or SEQ ID No. 18 or SEQ ID No. 19 or SEQ ID No. 20 or SEQ ID No. 21 or SEQ ID No. 22 or SEQ ID No. 23 or SEQ ID No. 25 or SEQ ID No. 26.

According to a particular embodiment, the genetically modified *Pseudomonas putida* strain comprises a vector, in particular a plasmid, more particularly a plasmid comprising or having or consisting of the nucleotide sequence set forth in SEQ ID No. 31 or SEQ ID No. 33 or SEQ ID No. 4, the vector thus comprising an additional recombinant gene encoding a recombinant PKS comprising or having or consisting of the nucleotide sequence set forth in SEQ ID No. SEQ ID No. SEQ ID No. 16 or SEQ ID No. 17 or SEQ ID No. 18 or SEQ ID No. 19 or SEQ ID No. 20 or SEQ ID No. 21 or SEQ ID No. 22 or SEQ ID No. 23 or SEQ ID No. 25 or SEQ ID No. 26.

According to this particular embodiment, the genetically modified strain is capable of converting feruloyl-CoA into vanillylidene acetone via the PKS enzyme.

According to this particular embodiment, the genetically modified strain is capable of converting coumaroyl-CoA to 4-hydroxybenzalketone via the PKS enzyme.

Thus, an object of the invention relates to a genetically modified strain of *Pseudomonas putida* characterized in that it is capable of expressing or expresses a recombinant gene encoding a PKS capable of producing vanillylidene acetone from feruloyl-CoA and/or producing 4-hydroxybenzalacetone from p-coumaroyl-CoA.

In a preferred embodiment, the *Pseudomonas putida* strain according to the present application is capable of producing vanillylidene acetone from feruloyl-CoA and/or producing 4-hydroxybenzalacetone from p-coumaroyl-CoA.

In another preferred embodiment, the *Pseudomonas putida* strain according to the present application is capable of producing zingerone from vanillylidene acetone.

In another preferred embodiment, the *Pseudomonas putida* strain according to the present application is capable of producing raspberry ketone from 4-hydroxybenzalacetone.

coumaroyl-CoA or 4-coumaroyl-CoA is a compound of formula (I):

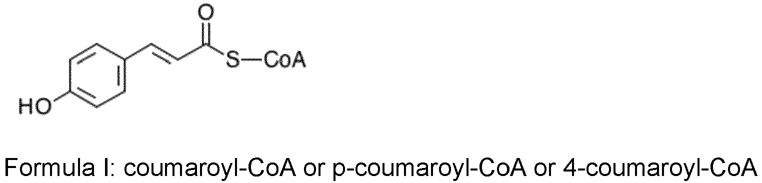

4-hydroxybenzalacetone is a compound of formula (II):

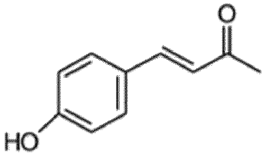

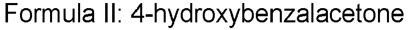

Raspberry ketone is a natural phenolic compound of IUPAC name 4-(4-Hydroxyphenyl)butan-2-one and of formula (III):

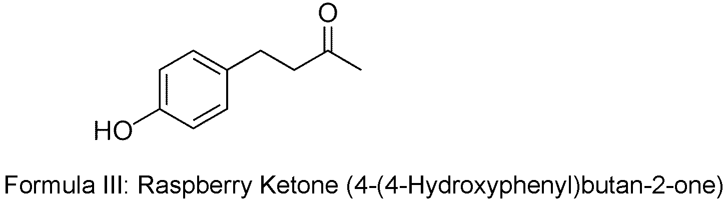

Ferulic acid or (2E)-3-(4-hydroxy-3-methoxyphenyl)prop-2-enoic acid (IUPAC name) is a compound of formula (IV):

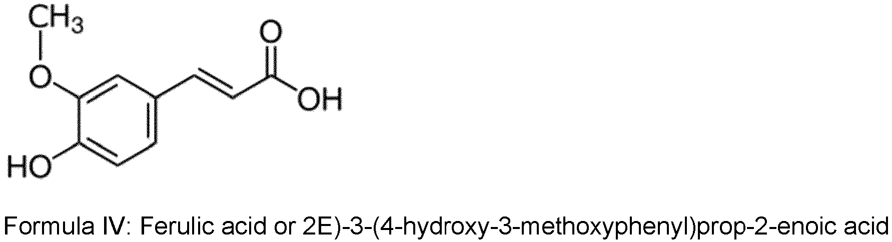

Feruloyl-CoA or trans-feruloyl-CoA(4-) is a precursor of Vanillylidene acetone and is a compound of formula (V):

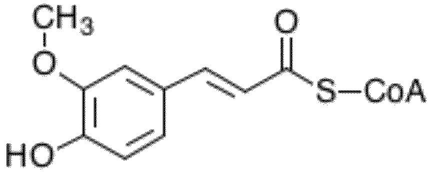

Vanillylidene acetone (Vda) or (E)-4-(4-hydroxy-3-methoxyphenyl)but-3-en-2-one (IUPAC name) (also referenced4-(4-hydroxy-3-methoxyphenyl)but-3-en-2-one herein) is a precursor of zingerone and is a compound of formula (VI):

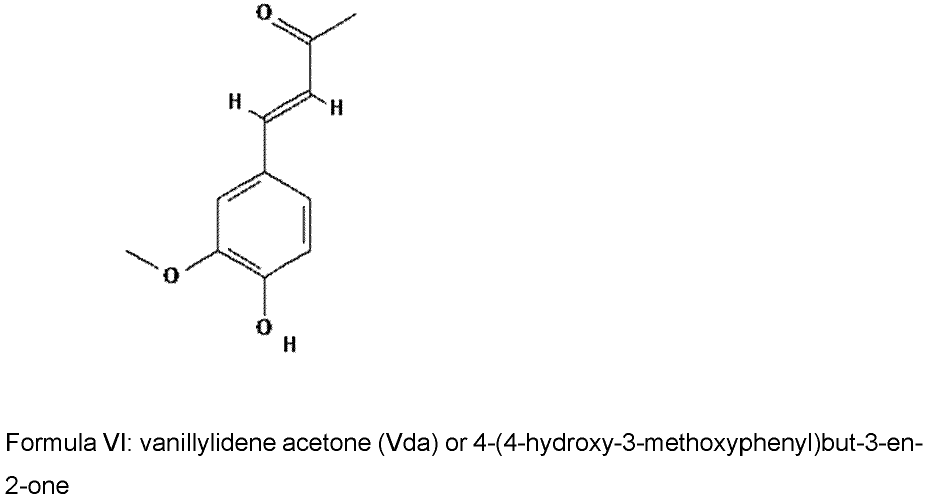

Zingerone, also called vanillylacetone, is a flavor component of ginger. Zingerone is also known under IUPAC name 4-(4-Hydroxy-3-methoxyphenyl)butan-2-one. Zingerone is a compound of formula (VII):

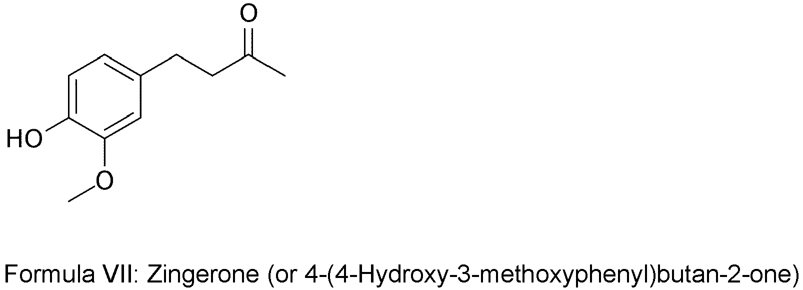

In another preferred embodiment, said genetically modified *Pseudomonas putida* strain is characterized in that it expresses a recombinant gene encoding a PKS according to the invention and at least one gene encoding a benzalacetone reductase, in particular selected from the group consisting of the NADPH-dependent 2-alkenal reductase of *Arabidopsis thaliana* (Uniprot Q39172, updated June 2, 202, the gene-reductase from *Zingiber officinale* (Uniprot A0A096LNF0, updated on April 7, 2021), the NADPH-dependent curcumin reductase, also called CurA from *Pseudomonas putida* (Uniprot Q88K17, updated on December 2, 2020) NADP-dependent alkenal double bond reductase, also known as DBR from *Olimarabidopsis pumila* (Uniprot A0A1C9CX65, updated August 12, 2020), NADP(+)-dependent 2-alkenal reductase, also known as DBR from Nicotiana tabacum (Uniprot Q9SLN8 ; EC 1. 3.1.102), or the NADP(+)-dependent 2-alkenal reductase, also known as Red from Capsicum annuum (Uniprot A0A1U8GFY1, updated 10 February 2021).

In a particular embodiment, the invention relates to a genetically modified strain of *Pseudomonas putida* characterized in that it expresses a recombinant gene encoding a functional equivalent of a previously described PKS.

Wild type strains of *Pseudomonas. putida* KT2440 are available for example in the NBRC Strain Bank (National Institute of Technology and Evaluation Biological Resource center https://www.nite.go.jp/en/nbrc/, NBRC100650).

Furthermore, strains of *Pseudomonas putida* or *Pseudomonas taiwanensis* optimized for tyrosine production are known to the skilled person who will be able to use them as starting strains to obtain the genetically modified strains according to the present invention (Calero et al., ACS Synth Biol. 2016 Jul 15;5(7):741-53; Wierckx et al, Appl Environ Microbiol. 2005 Dec;71(12):8221-7, Appl Environ Microbiol. 71(12):8221-7; Wynands et al, 2018; Otto et al 2019, Front Bioeng Biotechnol Nov 20;7:312).

As used in this description, the terms "genetically modified (*Pseudomonas putida*) strain," "modified (*Pseudomonas putida*) strain," or "genetically modified strain," are considered synonymous with each other.

In particular, "genetically modified strain" is understood to mean a strain that comprises either (i) at least one recombinant nucleic acid, or transgene, stably integrated into its genome, and/or present on a vector, e.g., a plasmid vector, or (ii) one or more unnatural mutations by nucleotide insertion, substitution, or deletion, said mutations being obtained by transformation techniques or by gene editing techniques known to the skilled person. In a particular embodiment, a genetically modified strain is a strain having stably integrated into its genome at least one exogenous nucleic acid, *i.e*., not naturally present in *P. putida,* for example a nucleic acid from another species, for example from *Rheum palmatum, Wachendorfia thyrsiflora, Rubus ideaus or Polygonum cuspidatum.*

For the purposes of the present invention, "recombinant gene encoding a PKS " means an exogenous nucleic acid comprising at least a portion encoding a benzalacetone synthase according to the invention as described above. In addition to the region encoding the PKS, the recombinant gene may be under the control of a promoter enabling its expression in the strain, preferably a promoter enabling its expression in the *P. putida* strain.

In a particular embodiment, the nucleic acid encoding a PKS according to the invention comprises a nucleotide sequence according to SEQ ID No. SEQ ID No. 16 or SEQ ID No. 17 or SEQ ID No. 18 or SEQ ID No. 19 or SEQ ID No. 20 or SEQ ID No. 21 or SEQ ID No. 22 or SEQ ID No. 23 or SEQ ID No. 25 or SEQ ID No. 26.or a sequence having at least 80%, 85%, 90%, 95% and most preferably at least 98% identity with a sequence of SEQ ID No. SEQ ID No. 16 or SEQ ID No. 17 or SEQ ID No. 18 or SEQ ID No. 19 or SEQ ID No. 20 or SEQ ID No. 21 or SEQ ID No. 22 or SEQ ID No. 23 or SEQ ID No. 25 or SEQ ID No. 26.

In one embodiment, which may be combined with the preceding ones, the recombinant gene encoding PKS as described above is placed under the control of a heterologous promoter, in particular a constitutive or inducible promoter, for example selected from among the ptrc, xyls/pm or araC/pBAD promoters, which allows the recombinant gene encoding PKS to be overexpressed in the genetically modified strain according to the invention.

The techniques of genetic modification by transformation, mutagenesis or gene editing are well known to the person skilled in the art and are described, for example, in "Molecular cloning: a laboratory manual", J. Sambrook, ed. Cold Spring Harbor, "Strategies used for genetically modifying bacterial genome: site-directed mutagenesis, gene inactivation," Journal of Zhejiang Univ-Sci B (Biomed & Biotechnol) 2016 17(2):83-99. and in Martinez-Garcia and de Lorenzo, "Pseudomonas putida in the quest of programmable chemistry," Current Opinion in Biotechnology, 59:111-121, 2019.

In one embodiment, a genetically engineered strain may comprise an expression-modifying nucleic acid, preferably overexpressing the expression of one or more genes naturally expressed in *Pseudomonas putida.*

Overexpression of a gene is understood as a higher expression of said gene in a genetically modified strain compared to the same strain in which the gene is expressed only under the control of the natural promoter. Overexpression can be achieved by inserting one or more copies of the gene directly into the genome of the strain, preferably under the control of a strong promoter, or also by cloning into plasmids, in particular multicopy plasmids, preferably also under the control of a strong promoter.

In another particular mode, a genetically modified strain may comprise a nucleic acid encoding one or more enzymes not naturally expressed in *Pseudomonas putida.*

Most advantageously, the Applicant has developed a *Pseudomonas putida* strain capable of expressing a PKS and efficiently producing vanillylidene acetone.

Most advantageously, the Applicant has developed a *Pseudomonas putida* strain capable of expressing a PKS and efficiently producing 4-hydroxybenzalacetone.

Preferably, the *Pseudomonas putida* strain according to the present disclosure is capable of overproducing zingerone from vanillylidene acetone.

Preferably, the *Pseudomonas putida* strain according to the present disclosure is capable of overproducing a raspberry ketone from 4-hydroxybenzalacetone.

Preferably, the *Pseudomonas putida* strain according to the present disclosure is capable of overproducing vanillylidene acetone from feruloyl-CoA.

Preferably, the *Pseudomonas putida* strain according to the present disclosure is capable of overproducing 4-hydroxybenzalacetone from coumaroyl-CoA.

Preferably, the *Pseudomonas putida* strain according to the present disclosure is capable of overproducing zingerone from feruloyl-CoA.

Preferably, the *Pseudomonas putida* strain according to the present disclosure is capable of overproducing raspberry ketone from coumaroyl-CoA.

In a particular embodiment of the invention, there is provided an overproducing strain of 4-hydroxybenzalacetone and/or raspberry ketone or vanillylidene acetone and/or zingerone. By "overproducing strain" in the sense of the present disclosure is meant a modified *Pseudomonas putida* strain capable of producing vanillylidene acetone and/or 4-hydroxybenzalacetone and/or a raspberry ketone or and/or zingerone in a higher amount compared to a wild type *Pseudomonas putida* strain.

Preferably, the overproducing strain according to the present disclosure is capable of producing 2, 3, 4, 5 or 6 times more vanillylidene acetone or 4-hydroxybenzalacetone and/or raspberry ketone or and/or zingerone as compared to a wild type *Pseudomonas putida* strain or a *Pseudomonas putida* strain expressing a wild type BAS as shown in the examples.

Preferably, the overproducing strain is capable of converting all of the feruloyl-CoA in situ or added to the culture medium to vanillylidene acetone, and preferably all the vanillylidene acetone into zingerone.

Preferably, the overproducing strain is capable of converting all of the coumaroyl-CoA in situ or added to the culture medium, to 4-hydroxybenzalacetone, and preferably converting all of the 4-hydroxybenzalacetone to raspberry.

According to another particular embodiment, the strain according to the present application is capable of producing raspberry ketone from 4-hydroxybenzalacetone or zingerone from vanillylidene acetone (*in situ* synthesis). The strain may thus further comprise at least one additional recombinant gene enabling the synthesis of raspberry ketone or zingerone, preferably a recombinant gene encoding a benzalacetone reductase.

By the term "additional recombinant gene" is meant in the sense of the present invention any recombinant gene present in the *Pseudomonas putida* strain in addition to the recombinant gene encoding a PKS according to the invention.

The additional recombinant gene may result from the insertion of a heterologous promoter, for example a strong promoter to overexpress an endogenous *Pseudomonas putida* gene, or a recombinant coding sequence encoding a protein not naturally expressed in *Pseudomonas putida.*

According to a particular embodiment, the genetically modified *Pseudomonas putida* strain comprises an additional recombinant gene encoding a tyrosine ammonia lyase (TAL) active polypeptide. A recombinant gene encoding TAL can be derived from the microorganism *Rhodotorula glutinis* and optimized according to reference Zhou et al, Appl Microbiol Biotechnol. 2016 Dec;100(24):10443-10452 (three-point mutations in this TAL enzyme make it more efficient: S9N; A11T; E518V). This TAL enzyme is called TAL_rg_opt. In particular, the modified strain may comprise a recombinant gene encoding a tyrosine ammonia lyase (TAL) (EC 4.3.1.23).

According to this particular embodiment, the genetically modified strain according to the invention is capable of converting tyrosine to coumaric acid via the TAL enzyme.

According to another particular embodiment that can be combined with the previous one, the genetically modified strain of *Pseudomonas putida* comprises an additional recombinant gene encoding a Feruloyl CoA synthase and/or a 4-coumarate-CoA ligase (4-CL). In particular, the modified strain may comprise a recombinant gene encoding a 4-CL (EC 6.2.1.12).

According to this particular embodiment, the genetically modified strain is capable of converting coumaric acid to p-coumaroyl-CoA via the 4-CL enzyme.

According to this particular embodiment, the genetically modified strain is capable of converting ferulic acid to feruloyl-CoA via the Feruloyl CoA synthase.

According to a preferred embodiment, the genetically modified *Pseudomonas putida* strain comprises one or several additional recombinant gene(s), namely one, two or the three following additional recombinant genes:
- a recombinant gene encoding a tyrosine ammonia lyase (TAL), and/or
- a recombinant gene encoding a 4-coumarate-CoA ligase (4-CL), and/or a Feruloyl CoA synthase (FcS); and/or
- a recombinant gene coding for a benzalacetone reductase (BAR).

The TAL, 4-CL, FcS, and BAR enzymes are all enzymes involved in the synthesis of phenylpropanoid compounds. According to this preferred embodiment, the modified strain is capable of producing a multitude of phenylpropanoid compounds, namely coumaric acid, p-coumaroyl-CoA, 4-hydroxybenzalcetone, and raspberry ketone or feruloyl-CoA, vanillylidene acetone and zingerone.

Another object of the invention relates to the use of a recombinant PKS according to the invention for producing vanillylidene acetone, zingerone, or vanillylidene acetone and zingerone.

Another object of the invention relates to the use of a recombinant PKS according to the invention for producing 4-hydroxybenzalcetone, or raspberry ketone, or 4-hydroxybenzalcetone and raspberry ketone.

According to a particular embodiment of the invention, it is provided a genetically modified strain of *Pseudomonas putida* as defined above, for use in the synthesis of vanillylidene acetone, or zingerone, or vanillylidene acetone and zingerone.

According to a particular embodiment of the invention, it is provided a genetically modified strain of *Pseudomonas putida* as defined above, for use in the synthesis of 4-hydroxybenzalcetone, or raspberry ketone, or 4-hydroxybenzalcetone and raspberry ketone.

Another object of the invention relates to a process for the synthesis of 4 vanillylidene acetone, or zingerone, or vanillylidene acetone and zingerone.

Another object of the invention relates to a process for the synthesis of 4-hydroxybenzalcetone, or raspberry ketone, or 4-hydroxybenzalcetone and raspberry ketone.

The process according to the invention comprises the implementation of a step of growth of a genetically modified strain of *Pseudomonas putida* as defined above in a culture medium under conditions allowing the expression of the recombinant gene encoding the benzalacetone synthase.

Preferably, the culture conditions are the culture conditions conventionally used in a fermenter for growth of *Pseudomonas putida.*

In a particular embodiment, the method according to the present disclosure is characterized in that the culture medium comprises ferulic acid, feruloyl-CoA, or coumaroyl-CoA.

In another embodiment, the process according to the present disclosure comprises carrying out a step of growing a genetically modified strain of *Pseudomonas putida* as defined above in a culture medium under conditions that allow for the expression of the recombinant gene encoding benzalacetone synthase and one or several additional recombinant gene(s) necessary to synthesize raspberry ketone or zingerone starting from L-Tyrosine or coumarate or coumaroyl-CoA or ferulic acid.

According to a particular embodiment, the process comprises a step of growing a genetically modified strain of *Pseudomonas putida* comprising a recombinant gene encoding a benzalacetone synthase according to the invention and comprising the one, two or the three following additional recombinant genes encoding:
- a tyrosine ammonia lyase (TAL), and/or
- a 4-coumarate-CoA ligase (4-CL), and/or feruloyl-CoA (FcS); and/or
- a benzalacetone reductase (BAR).

According to a particular embodiment, the synthesis process according to the invention allows the production of raspberry ketone or zingerone in large quantities, for example in yields of at least 100 mg/L, preferably at least 500 mg/L, more preferably at least 1 g/L, 1.5 g/L or 2 g/L and in particular in 2-fold, 3-fold, 4-fold, 5-fold or 6-fold production quantities in comparison with a wild type *Pseudomonas putida* strain or one comprising the following additional recombinant genes encoding :
- a tyrosine ammonia lyase (TAL),
- a 4-coumarate-CoA ligase (4-CL), or feruloyl-CoA (FcS),
- a benzalacetone reductase (BAR).

In one embodiment, the process converts at least 50%, preferably 60%, 70%, 80%, 90%, 95%, or even at least 99% of the feruloyl-CoA or coumaroyl-CoA synthesized *in situ* by the strain or added within the culture medium of the strain into vanillylidene acetone or 4-hydroxybenzalcetone, respectively.

The synthesis process according to the invention may also comprise a step of purification and/or recovery of the vanillylidene acetone and/or zingerone or the 4-hydroxybenzalcetone and/or raspberry ketone product by the strain.

The present invention also concerns a zingerone or raspberry ketone obtained by the processes and methods disclosed herein, in particular by using a recombinant PKs as disclosed here above.

### Examples

### Example 1: in silico identification of modified PKS

In the literature, it has been demonstrated that a PKS from *Piper methysticum* (PmPKS) may achieve higher zingerone production than a BAS enzyme from *Rheum palmatum* (Construction of an Artificial Biosynthetic Pathway for Zingerone Production in Escherichia coli Using Benzalacetone Synthase from *Piper methysticum;* Kyung Taek et al. 2021).

On the basis of these results, the inventors selected wild type PKS enzymes for improving the overall production of zingerone in bacteria. In addition to the PKS from *Piper methysticum* (PmPKS), 2 other PKS with sequence similarities with the PmPKS enzyme were selected: the EgPKS gene from *Elaeis guineensis* and the VvPKS gene from *Vitis vinifera.* These PKS share respectively 86% and 84% sequence homology with the sequence of PmPKS. These sequences of the wild type PKSs can be retrieved from public databases such as Uniprot and NCBI. These enzymes were categorized as being PKS due to their high homology, but their activity on the degradation of ferulic acid was not known, neither their capability to catalyze the transformation of feruloyl-CoA into vanillylidene acetone. Wild type PmPKS corresponds to the protein with amino acid sequence set forth in SEQ ID No. 1; EgPKS corresponds to the protein with amino acid sequence set forth in SEQ ID No. 2; VvPKS corresponds to the protein with amino acid sequence set forth in SEQ ID No. 3.

First, the inventors tested the *in vivo* activity of these PKS enzymes (PmPKS, VvPKS and EgPKS) on ferulic acid. They were able to confirm that the selected PSK were able to transform feruloyl-CoA into vanillylidene acetone (see example 2).

With the aim to improve the activity on these PKS, the inventors optimized *in silico* the sequence of each PKS. It has been shown that the stability of an enzyme can be correlated with its catalytic efficiency. Based on this principle, Goldenzweig, A., et al. (2016) provided a protocol that estimates the free energy of an enzyme and suggests mutations to improve its stability/efficiency. A modified version of this protocol has been used for identifying modified PKS with mutations that might enhance the stability of the PKS issued from *Piper methysticum, Elaeis guineensis,* and *Vitis vinifera.*

This adapted protocol can be seen as a two-step process wherein:
In a first step, a sequence alignment with homologous proteins is performed. Rather than selecting the most promising mutations for each position, this step is performed to eliminate rare or unobserved mutations. A "position specific scoring matrix" is calculated that allow selecting mutations with a positive score.
In a second step, ROSETTA software is used to evaluate potentially stabilizing point mutations, selecting only those that result in a decrease in energy as compared to the wild-type enzyme. For each iteration, an amino acid is mutated, and the energy difference is calculated is performed. The mutations associated with a negative variation in energy are kept. Before or after the second step, an intermediate filter involving the elimination of unreasonable mutations by manual inspection can be performed. In particular, residues with hydrophobic chains exposed to the solvent, prolines introduced inside the alpha helix (Rigoldi, F., et al. 2018) or sites known to be active or responsible for a desired activity are deleted from the potentiate mutations.

At the end of the first step, approximately 490 common mutations according to the natural diversity were identified. After the second step, approximately 50 positions were selected for modification, with between 1 and 3 possible mutations for each position, for a total of 73 individual mutations that might enhance stability of the PKS enzymes. Among these, 20 mutations were selected; those with the best energic score (the more stabilizing the mutation are those with the more negative the score). It should be noted that any mutated BAS disclosed in the following table may be part of the present invention, depending on its catalytic activity on the transformation of feruloyl-CoA into vanillylidene acetone as compared to the wild-type protein from which it is issued. The link between the mutation and the energic score is illustrated in the three following tables (each table represent this link for a PKS issued from a specific species).

**Table 1: List of mutated PKS as compared to wild type PmPKS and calculated energic score.**

| Mutations | Score ΔΔG |
|---|---|
| P38A | -5.63869 |
| V322P | -3.98223 |
| M65Q | -3.37834 |
| S221A | -2.67232 |
| V322K | -2.19918 |
| V322E | -2.17235 |
| M50L | -1.89192 |
| V322A | -1.85622 |
| V20I | -1.84025 |
| T158F | -1.71239 |
| D252E | -1.69638 |
| T158V | -1.47964 |
| T158Y | -1.38227 |
| A183R | -1.37389 |
| M210L | -1.35465 |
| A29S | -1.27118 |
| A29E | -1.24521 |
| V232S | -1.23511 |
| R319N | -1.13899 |

**Table 2: List of mutated PKS as compared to wild type EqPKS and calculated energic score.**

| **Mutations** | Score ΔΔG |
|---|---|
| V290A | -9.48426 |
| **P39A** | -5.47575 |
| **E16D** | -2.21225 |
| **M66Q** | -1.79303 |
| **K323P** | -1.43386 |
| **A30E** | -1.20365 |
| **S295T** | -1.09962 |
| **I234V** | -1.07243 |
| **S284A** | -1.04585 |
| **K47N** | -0.971822 |
| **E56K** | -0.94406 |
| **A30D** | -0.92409 |
| **H387R** | -0.828485 |
| **S75T** | -0.71084 |
| **D257A** | -0.706981 |
| **V290K** | -0.634358 |
| **L6I** | -0.627031 |
| **A361K** | -0.613698 |
| **A30S** | -0.562098 |

**Table 3: List of mutated PKS as compared to wild type VvPKS and calculated energic score.**

| **Mutations** | Score ΔΔG |
|---|---|
| K288A | -8.08615 |
| **P37A** | -5.24233 |
| **M64Q** | -3.25393 |
| **G5E** | -2.94242 |
| **G359K** | -2.59312 |
| **A388P** | -2.53491 |
| **G359A** | -2.23136 |
| **L247I** | -2.17037 |
| **G5D** | -2.11129 |
| **G359S** | -1.86633 |
| **M49K** | -1.56632 |
| **H385R** | -1.53426 |
| **L247V** | -1.4549 |
| **A182R** | -1.38731 |
| **M49L** | -1.37245 |
| **G216S** | -1.36601 |
| **A28E** | -1.36294 |
| **G359T** | -1.27453 |
| **K2315** | -1.1784 |

For each PKS, three mutations that might confer the best stabilization of the enzyme were selected for validation *in vivo* (see example 3). For the PmPKS, mutants with single point mutation P38A (having the amino acid sequence set forth in SEQ ID No. 4, encoded by the nucleotide sequence set forth in SEQ ID No. 19, and encoded within the vector of SEQ ID No. 35), V322P (having the amino acid sequence set forth in SEQ ID No. 5, encoded by the nucleotide sequence set forth in SEQ ID No. 20, and encoded within the vector of SEQ ID No. 36), or M65Q (having the amino acid sequence set forth in SEQ ID No. 12, encoded by the nucleotide sequence set forth in SEQ ID No. 27, and encoded within the vector of SEQ ID No. 37) were selected. For the EgPKS, mutants with single point mutation V290A (having the amino acid sequence set forth in SEQ ID No. 13, encoded by the nucleotide sequence set forth in SEQ ID No. 28, and encoded within the vector of SEQ ID No. 44), P39A (having the amino acid sequence set forth in SEQ ID No. 6, encoded by the nucleotide sequence set forth in SEQ ID No. 21, and encoded within the vector of SEQ ID No. 45) or E16D (having the amino acid sequence set forth in SEQ ID No. 14, encoded by the nucleotide sequence set forth in SEQ ID No. 29, and encoded within the vector of SEQ ID No. 46) were selected. For the VvPKS, mutants with single point mutation K288A (having the amino acid sequence set forth in SEQ ID No. 15, encoded by the nucleotide sequence set forth in SEQ ID No. 30, and encoded within the vector of SEQ ID No. 39), P37A (having the amino acid sequence set forth in SEQ ID No. 7, encoded by the nucleotide sequence set forth in SEQ ID No. 22, and encoded within the vector of SEQ ID No. 40) or M64Q (having the amino acid sequence set forth in SEQ ID No. 8, encoded by the nucleotide sequence set forth in SEQ ID No. 23, and encoded within the vector of SEQ ID No. 41) were selected.

### In vivo experimentations (examples 2 and 3): Materials and methods

### 1- Mutagenesis of the gene coding for the PKSs of Piper methysticum, Elaeis guineensis, and Vitis vinifera.

Polynucleotides coding for the gene of the PKSs of *Piper methysticum, Elaeis guineensis,* and *Vitis vinifera* are each cloned into the plasmid pBBR1-MCS2 (Kovach et al., 1995; having the sequence of SEQ ID No. 31 or SEQ ID No. 33 or SEQ ID No. 42) downstream of the ptrc promoter which allows the overexpression of the gene in Pseudomonas putida KT2440.

Directed mutagenesis of these genes is performed with PCR primers containing the desired mutation so as to provide a plurality of genes, each encoding a mutated PKS enzyme as selected after the *in silico* identification).

Cloning and site-directed mutagenesis are performed in *E*. *coli* S17.1 strain.

### 2- Bacterial transformation

The expression plasmids of the wild type PKS (plasmid pC2F004, of SEQ ID No. 34 for Wild type PmPKS, of SEQ ID No. 38 for VvPKS, and of SEQ ID No. 43 for EgPKS), and of the different mutated PKSs (of SEQ ID No. 35; SEQ ID No. 36; SEQ ID No. 37; SEQ ID No. 39; SEQ ID No. 40; SEQ ID No. 41; SEQ ID No. 44; SEQ ID No. 45; SEQ ID No. 46) are transformed in the bacteria *Pseudomonas putida* KT2440 ppu_310 (strain which has all the enzymes for the production of zingerone starting from ferulic acid). The strain genotype is referenced ΔPP_3358 ΔphaA::P14g_RBS007_Red_Ca ΔpykA::P14g_RBS007_4-Cl_At.

As control, the bacteria *Pseudomonas putida* is transformed with plasmid (of SEQ ID No. 32) comprising a gene encoding BAS (of SEQ ID No. 24) from *Rheum palmatum,* which is thus able to convert feruloyl-Coa into VDA and coumaroyl-CoA into 4-hydroxybenzalacetone.

### 3- Culture and production of zingerone

The different modified strains of recombinant *P. putida* are cultured in 1 mL of MPpu medium + 5g/L Glucose + ferulic acid (0.5mM) with seeding at OD₆₀₀ = 0.05. The cultures are placed at 30°C, under agitation 140 rpm for 48h. At the end of 48h, the cultures are centrifuged, the supernatants are recovered for HPLC assay.

### 4- HPLC assay

The concentration of molecules of ferulic acid (FA), vanillylidene acetone (VDA) and zingerone (ZGO) present in the supernatant are determined by HPLC. The sample is run through HPLC with a Kinetex 5 µm F5 100Å column (Phenomenex) with 0.1% formic acid with acetonitrile gradient as mobile phase. The analysis method lasts 35min. The oven temperature is 40°C and the pump flow rate is 1mL/min. Detector used: UV (DAD) 280 nm, UV (DAD) 315 nm. Injection volume: 10µL. The analysis is run according to the following gradient:

**Table 4: Analysis method gradient**

| Time (min) | % acetonitrile |
|---|---|
| 0 | 0 |
| 5 | 0 |
| 25 | 25 |
| 30 | 38 |
| 35 | 0 |

### Example 2: Production of vanillylidene acetone and zingerone from ferulic acid in recombinant P. putida expressing wild type PKS from Piper methysticum, Elaeis guineensis, and Vitis vinifera or wild type BAS from Rheum palmatum.

### Results

On **figure 1**, middle and right panels, are illustrated the concentrations of VDA and ZGO, obtained from the transformation of feruloyl-CoA (consumption of ferulic acid is illustrated on **figure 1**, left panel). The wild type PmPKS enzyme has a better activity on vanillylidene acetone and zingerone production, as compared to the activity of the BAS from *Rheum palmatum* on the production of the same compounds. It can be seen that in presence of PmPKS, zingerone concentration in the medium is around 30µM while the concentration of zingerone is approximately of 4µM when the conversion of feruloyl-CoA into VDA is catalyzed by the BAS from *R. palmatum.* As illustrated, the cultured *P. putida* transformed with a wild type PKS leads to the production of large amount of feruloyl-CoA and ZGO, illustrating their higher capability for transforming feruloyl-CoA into VDA and then ZGO. All three wild PKS allow a better yield for producing zingerone than any other recombinant bacteria. Further, VvPKS allow the consumption of almost all ferulic acid, leaving only VDA (30µM) and ZGO (13µM) in the culture medium. With the EgPKS enzyme, even if ferulic acid remains in the culture medium, the ferulic acid that has been consumed has been converted into VDA (13µM) and ZGO (19µM).

Under these conditions, PmPKS, EgPKS and VvPKS are all enzyme that allow the production of higher yield of ZGO, as compared to prior art solutions. These enzymes all show interesting characteristics for producing ZGO, more efficient than the RpBAS enzyme.

These results clearly illustrate the higher efficiency of *P. putida* strains transformed with the wild type PKS according to the invention for producing VDA and ZGO as compared to P. putida strain transformed with wild-type BAS. The wild type PKS allows Pseudomonas putida to consume ferulic acid for producing better amount of VDA and zingerone, way over the yield obtained with other strains transformed with BAS for example.

### Example 3: Production of vanillylidene acetone and zingerone from ferulic acid in recombinant P. putida expressing mutated PKS according to the invention.

### Results

On **figure 2** are illustrated the consumption of ferulic acid (left panel), the production of VDA (middle panel), and the production of Zingerone (right panel) by bacteria *P*. *Putida* transformed with genes encoding the mutated PKS of the invention. It can be seen that the different mutations inserted into the wild type PKSs lead to different efficiency of the mutated PKS for producing zingerone.

For PmPKS, the M65Q mutation gives similar results to PmPKS Wt. The P38A and V322P mutations give higher VDA concentrations, 40µM and 60µM respectively. Further, the V322P mutation seems to enable higher ZGO production (63µM).

For EgPKS, mutations V290A and E16D have a deleterious effect. Indeed, with these two mutants, no production of VDA and ZGO has been observed. The P39A mutant shows nonetheless an improvement in the production of these two compounds. A production of 68µM VDA and 36µM ZGO was reached with this mutated PKS.

For VvPKS, the K288A mutation has a negative effect on the enzyme. The latter results in an absence of VDA and ZGO production under these conditions. The P37A and M64Q mutations improve VDA (x3) and ZGO (x7.7) production as compared with the wild type enzyme. We observe a production of around 90µM of VDA and around 100µM of ZGO. The P37A mutant appears to be the most efficient for ZGO production.

These results clearly illustrate the higher efficiency of *P. putida* strains transformed with the mutated PKS according to the invention for producing VDA and ZGO as compared to P. putida strain transformed with wild type. The mutated PKS allows Pseudomonas putida to consume ferulic acid for producing better amount of VDA and zingerone, way over the yield obtained with other strains transformed with BAS for example.

### Example 4: Production of 4-HBA and raspberry ketone in recombinant P. putida expressing mutated PKS according to the invention.

### Materials and Methods

Mutagenesis (step 1), bacterial transformation (step 2) and HPLC analysis (step 4) are identical as those presented for example 2 and 3. The step of culturing transformed bacteria for producing raspberry ketone is the following!

### 3- Raspberry ketone production

The different modified strains of *P*. *putida* constructed are cultured in 10mL of MPpu medium + 5g/L Glucose + p-coumaric acid (0.5mM) with seeding at OD₆₀₀ = 0.05.

The cultures are placed at 30°C, under agitation 140 rpm for 48h. At the end of 48h, the cultures are centrifuged, the supernatants are recovered for HPLC assay.

### Results

As illustrated on **figure 3****,** left panel, the concentration of pCA (4-coumaroyl-CoA) is low in cultured *P. putida* transformed with a wild type PmPKS, EgPKS, VvPKS, or with the mutated PKS according to the invention (those which have a mutation as defined in claim 1). The pCA in *P. putida* cells transformed with these PKS is either absent or broadly consumed as compared to P. Putida cells transformed with *Rheum palmatum* BAS. This means that pCa has been consumed by strains of P. Putida transformed with wild type PKs or mutated PKS according to the invention. In other medium containing strains transformed with other mutated PKS or with BAS, pCA may be found in large quantities. The PKS enzymes of the invention are thus more efficient than other to catalyze the transformation of pCA.

On figure 3, middle and right panels, are illustrated the concentrations of HBA (4-hydroxybenzalacetone) and FBO (raspberry ketone), obtained from the transformation of 4-coumaroyl-CoA (pCA). As illustrated, the cultured *P. putida* transformed with wild type PmPKS, EgPKS, VvPKS, or with the mutated PKS according to the invention lead to the production of large amount of HBA and FBO, illustrating their higher capability for transforming pCA into HBA and then FBO.

As illustrated on **figure 3****,** the mutated PKS allows *Pseudomonas putida* to consume almost all of the PCA and produce up to 0.075 mM of HBA and 0.035 mM of raspberry ketone, way over the yield obtained with other strains. The *P. putida* strains with the wild type PKS or the mutated PKS of the invention are more efficient than prior art strains for transforming pCA into HBA, and *in fine* for producing raspberry ketone.

These results clearly illustrate the higher efficiency of *P. putida* strains transformed with wild type PKS or the mutated PKS of the invention for producing HBA and *in fine* FBO as compared to P. putida strain transformed with wild type BAS.

### List of cited documents

### Patent literature

### Non-patent literature

Abe, Ikuro, et al. « Benzalacetone Synthase: A Novel Polyketide Synthase That Plays a Crucial Role in the Biosynthesis of Phenylbutanones in Rheum palmatum ». European Journal of Biochemistry, vol. 268, no 11, juin 2001, p. 3354-59. DOI.org (Crossref), https:/Idoi.org/10.1046/j.1432-1327.2001.02255.x.

Abe, Tsuyoshi, et al. « Structure Function Analysis of Benzalacetone Synthase from Rheum palmatum ». Bioorganic & Medicinal Chemistry Letters, vol. 17, no 11, juin 2007, p. 3161-66. ScienceDirect, https://doi.org/10.1016/j.bmcl.2007.03.029.

Chang, Chen, et al. « Efficient Bioconversion of Raspberry Ketone in Escherichia Coli Using Fatty Acids Feedstocks ». Microbial Cell Factories, vol. 20, no 1, mars 2021, p. 68. PubMed, https://doi.org/10.1186/s12934-021-01551-0.

Goldenzweig, Adi, et al. « Automated Structure- and Sequence-Based Design of Proteins for High Bacterial Expression and Stability ». Molecular Cell, vol. 63, no 2, juillet 2016, p. 337-46. PubMed, https://doi.org/10.1016/j.molcel.2016.06.012.

Häkkinen, Suvi T., et al. « Bioconversion to Raspberry Ketone is Achieved by Several Non-related Plant Cell Cultures ». Frontiers in Plant Science, vol. 6, novembre 2015. PubMed Central, https://doi.org/10.3389/fpls.2015.01035.

Kyung Taek et al. , Construction of an Artificial Biosynthetic Pathway for Zingerone Production in Escherichia coli Using Benzalacetone Synthase from Piper methysticum. J Agric Food Chem 2021 Dec 8;69(48):14620-14629.

Kovach, M. E., et al. « Four New Derivatives of the Broad-Host-Range Cloning Vector PBBR1MCS, Carrying Different Antibiotic-Resistance Cassettes ». Gene, vol. 166, no 1, décembre 1995, p. 175-76.

Lee, Danna, et al. « Heterologous production of raspberry ketone in the wine yeast Saccharomyces cerevisiae via pathway engineering and synthetic enzyme fusion ». Microbial Cell Factories, vol. 15, mars 2016. PubMed Central, https://doi.org/10.1186/s12934-016-0446-2.

Liu, Chang, et Sijin Li. « Engineered Biosynthesis of Plant Polyketides by Type III Polyketide Synthases in Microorganisms ». Frontiers in Bioengineering and Biotechnology, vol. 10, 2022, p. 1017190. PubMed, https://doi.org/10.3389/fbioe.2022.1017190.

Milke, Lars, et al. « Synthesis of the Character Impact Compound Raspberry Ketone and Additional Flavoring Phenylbutanoids of Biotechnological Interest with Corynebacterium Glutamicum ». Microbial Cell Factories, vol. 19, no 1, avril 2020, p. 92. PubMed, https://doi.org/10.1186/s12934-020-01351-y.

Morita, H., et al. « A Structure-Based Mechanism for Benzalacetone Synthase from Rheum palmatum. » Proceedings of the National Academy of Sciences of the United States of America, vol. 107, no 2, janvier 2010, p. 669-73. europepmc.org, https://doi.org/10.1073/pnas.0909982107.

Rigoldi, Federica, et al. « Review: Engineering of Thermostable Enzymes for Industrial Applications ». APL Bioengineering, vol. 2, no 1, mars 2018, p. 011501. PubMed, https://doi.org/10.1063/1.4997367.

Shimokawa, Yoshihiko, et al. « Benzalacetone Synthase ». Frontiers in plant science, vol. 3, mars 2012. PubMed Central, https://doi.org/10.3389/fpls.2012.00057.

Wang, Chengcheng, et al. « Construction of Synthetic Pathways for Raspberry Ketone Production in Engineered Escherichia Coli ». Applied Microbiology and Biotechnology, mars 2019. Crossref, https://doi.org/10.1007/s00253-019-09748-5.

## Claims

1. A recombinant polyketide synthase (PKS) issued or derived from a wild type PKS from *Vitis vinifera* (VvPKS), *Piper methysticum* (PmPKS), or *Elaeis guineensis* (EgPKS), the recombinant PKS having a P37A substitution and/or a M64Q substitution as compared to the wild type PKS from *Vitis vinifera,* or the recombinant PKS having a P38A substitution, and/or a V322P substitution as compared to the wild type PKS from *Piper methysticum,* or the recombinant PKS having a P39A substitution as compared to the wild type PKS from *Elaeis guineensis.*

2. The recombinant PKS according to claim 1 which has an improved catalytic activity on the transformation of feruloyl-CoA into vanillylidene acetone and/or on the transformation of coumaroyl-CoA into 4-hydroxybenzalacetone, as compared to the wild type PKS from which it is issued or derived.

3. The recombinant PKS according to claim 1 or 2, which has at least the same catalytic activity, in particular at least the same catalytic constant k_{cat}, on the transformation of feruloyl-CoA into vanillylidene acetone and/or on the transformation of coumaroyl-CoA into 4-hydroxybenzalacetone as compared to at least one wild type PKS having the amino acid sequence set forth in SEQ ID No. 3, SEQ ID No. 2, or SEQ ID No. 1.

4. The recombinant PKS according to claim 3, wherein the catalytic activity of the recombinant PKS on the transformation of feruloyl-CoA into vanillylidene acetone and/or on the transformation of coumaroyl-CoA into 4-hydroxybenzalacetone is determined by measuring by HPLC the total concentration of feruloyl-CoA and/or vanillylidene acetone and/or coumaroyl-CoA and/or 4-hydroxybenzalcetone and/or raspberry ketone and/or zingerone, preferably feruloyl-CoA and vanillylidene acetone or coumaroyl-CoA and 4-hydroxybenzalcetone before and after a catalytic reaction of transforming feruloyl-CoA into vanillylidene acetone and/or on the transformation of coumaroyl-CoA into 4-hydroxybenzalacetone.

5. The recombinant PKS according to any one of claims 1 to 4, which has at least 90% identity to the wild type protein from which it is issued, in particular which has at least 90% identity with the PKS having the amino acid sequence set forth in SEQ ID No. 7 or SEQ ID No. 8 or SEQ ID No. 11 or SEQ ID No. 4 or SEQ ID No. 5 or SEQ ID No. 6 or SEQ ID No. 10.

6. The recombinant PKS according to any one of claims 1 to 5, which has the amino acid sequence set forth in SEQ ID No. 7, SEQ ID No. 8, SEQ ID No. 11, SEQ ID No. 4, SEQ ID No. 5, SEQ ID No. 6 or SEQ ID No. 10.

7. A nucleic acid molecule encoding the recombinant PKS according to any one of claims 1 to 6, comprising or consisting of a polynucleotide encoding a recombinant PKS according to claim 1, in particular a nucleic acid molecule comprising or consisting of the nucleotide sequence set forth in SEQ ID No. 19 or SEQ ID No. 20 or SEQ ID No. 21 or SEQ ID No. 22 or SEQ ID No. 23 or SEQ ID No. 25 or SEQ ID No. 26, or a vector, in particular a plasmid, comprising the nucleotide sequence set forth in SEQ ID No. 19 or SEQ ID No. 20 or SEQ ID No. 21 or SEQ ID No. 22 or SEQ ID No. 23 or SEQ ID No. 25 or SEQ ID No. 26.

8. A genetically modified *Pseudomonas putida* strain capable of expressing a wild type PKS from *Piper methysticum* (PmPKS), *Elaeis guineensis* (EgPKS), or *Vitis vinifera* (VvPKS) and/or a recombinant PKS as defined in any one of claims 1 to 6, in particular comprising a nucleic acid molecule encoding a wild type PKS from *Piper methysticum* (PmPKS), *Elaeis guineensis* (EgPKS), or *Vitis vinifera* (VvPKS) and/or a recombinant PKS as defined in any one of claims 1 to 6.

9. The genetically modified *Pseudomonas putida* strain according to claim 8, which produces vanillylidene acetone and/or zingerone, in particular from feruloyl-CoA; and/or which produces 4-hydroxybenzalacetone and/or a raspberry ketone, in particular from coumaroyl-CoA.

10. The genetically modified *Pseudomonas putida* strain according to claim 8 or 9, which comprises:
- a gene encoding a tyrosine ammonia lyase; and/or
- a gene encoding a 4-coumarate-ligase, and/or
- a gene encoding a benzalacetone reductase.

11. The genetically modified *Pseudomonas putida* strain according to claim 10, which comprises a gene encoding a tyrosine ammonia lyase; and/or a gene encoding a 4-coumarate-ligase and/or a gene encoding a Feruloyl CoA synthase, and/or a gene encoding a benzalacetone reductase.

12. Use of a PKS according to any one of claims 1 to 6, or a wild type PKS from *Vitis vinifera* (VvPKS) or from *Elaeis guineensis* (EgPKS) for producing raspberry ketone and/or zingerone, in particular by biosynthesis into a bacteria.

13. A method for producing vanillylidene acetone and/or zingerone, in particular from a feruloyl-CoA, or for producing 4-hydroxybenzalacetone and/or a raspberry ketone, in particular from a coumaroyl-CoA, comprising a step of using a PKS according to any one of claim 1 to 6, or a wild type PKS from *Vitis vinifera* (VvPKS) or from *Elaeis guineensis* (EgPKS), or a genetically modified *Pseudomonas putida* strain according to any one of claims 8 to 11.

14. A process for producing vanillylidene acetone and/or zingerone, in particular from a feruloyl-CoA, or for producing 4-hydroxybenzalacetone and/or a raspberry ketone, in particular from a coumaroyl-CoA, comprising a step of culturing a genetically modified *Pseudomonas putida* strain according to any one of claims 8 to 11, in condition allowing the expression of the recombinant gene encoding the recombinant PKS according to any one of claim 1 to 6, or a wild type PKS from *Vitis vinifera* (VvPKS) or from *Elaeis guineensis* (EgPKS).

15. A zingerone or a raspberry ketone obtained by the process of claim 14.
